(19) Europäisches Patentamt
European Patent Office
Office européen des brevets

(11) Publication number: **0 272 576 B1**

(12) # EUROPEAN PATENT SPECIFICATION

(45) Date of publication of patent specification: **27.02.91**

(51) Int. Cl.$^5$: **C07C 219/10**, A61K 7/44, A61K 7/06

(21) Application number: **87118494.1**

(22) Date of filing: **14.12.87**

(54) **Quaternary ammonium halide salts of cinnamate esters as sunscreen agents and compositions containing same.**

(30) Priority: **23.12.86 US 945914**

(43) Date of publication of application:
**29.06.88 Bulletin 88/26**

(45) Publication of the grant of the patent:
**27.02.91 Bulletin 91/09**

(84) Designated Contracting States:
**AT BE CH DE ES FR GB IT LI NL**

(56) References cited:
**EP-A- 0 165 710**
**DE-C- 957 162**
**FR-A- 2 504 530**

(73) Proprietor: **L. GIVAUDAN & CIE Société Anonyme**

**CH-1214 Vernier-Genève(CH)**

(72) Inventor: **Cohen, Isaac David**
**1885 East 13th Street**
**Brooklyn, N.Y. 11229(US)**

(74) Representative: **Urech, Peter, Dr. et al**
**Grenzacherstrasse 124 Postfach 3255**
**CH-4002 Basel(CH)**

**EP 0 272 576 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

A 1978 FDA monograph on over-the-counter sunscreens [Federal Register, Vol. 43, No. 166, August 25, 1978] indicates that risk factors of chronic exposure to the sun may include the development of keratoses, skin cancers, and premature aging of the skin. More recently, a review on the use of sunscreens in hair-care products [J.P. Pavlichko, Drug and Cosmetic Industry, 137 (6) 35 (1985)] states that "hair damage from exposure to sunlight can produce color fading, texture changes, reduction of water uptake, loss of elasticity, and keratin structure changes". These and other publications highlight an increasing awareness about the detrimental effects that extended exposure to the sun has on living and non-living tissue, as well as to commercial and industrial materials. It is generally accepted that these detrimental effects can be prevented or mitigated by the use of sunscreen agents which efficiently absorb ultraviolet radiation.

A successful sunscreen agent must not only be able to efficiently absorb the ultraviolet light which can cause damage to skin, hair, fabric, etc. but must also have other properties that make it compatible with the system in which it is being used. For example, skin care preparations such as astringents, hydroalcoholic suntan lotions and hydroalcoholic gels are generally formulated without any oily components. For such products, it is required that the UV absorber possess significant aqueous, alcoholic and/or hydroalcoholic solubility, and be, at the same time, resistant towards removal from the skin surface by washing, perspiration etc. The ability of the UV absorber to adhere to and be retained by the skin during swimming and during Activities causing perspiration, i.e., substantivity, is a highly desirable property in a cosmetic sunscreen. Similarly, it is also a highly desirable property for sunscreens used in hair conditioners and fabric softeners where it is also required that the sunscreen be capable of being incorporated into aqueous-based systems while at the same time be highly substantive to the hair or fabric to be protected.

For products such as hair conditioners, fabric softeners and skin care preparations there is a need for the development of new and unique UV absorbing molecules which have solubility characteristics that allow for formulation into alcoholic, hydroalcoholic and/or aqueous systems while at the same time providing high substantivity to the surface to be protected.

In accordance with the present invention there are provided halide salts of cationic cinnamate esters of the general formula

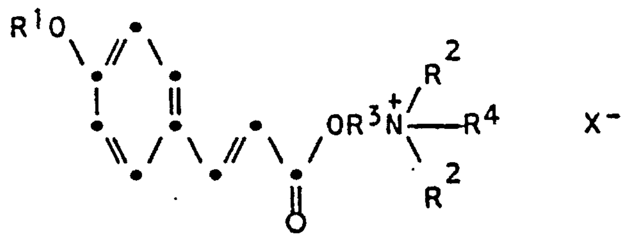

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is a halogen anion, e.g. fluoride, chloride, bromide or iodide.

It has now been found that these novel salts, which have a long chain alkyl group bonded to a quaternary nitrogen, are effective ultraviolet absorbers which possess an unexpectedly high degree of substantivity. p-Methoxy cinnamate esters containing quaternary ammonium groups in which the quaternary centers are substituted with alkyl groups have been reported. [See A. Berg et al., German Patent Specification 957,162, and L. Jung et al., French Patent Publication 2,504,530.*] Nothing reported in the prior art however, teaches nor suggests that the novel salts of this invention would have the unique combination of properties which allow them to be formulated effectively into alcoholic, hydroalcoholic, and/or aqueous compositions to provide sunscreen products which have superior substantivity.

The cationic cinnamate esters of this invention may be conveniently prepared via quaternization of suitably substituted tertiary amines II with alkylating agents III as shown below in Scheme I,

* whereby these two patent specifications also exhibit Examples of corresponding cosmetic formulations, and EP-A-165 710.

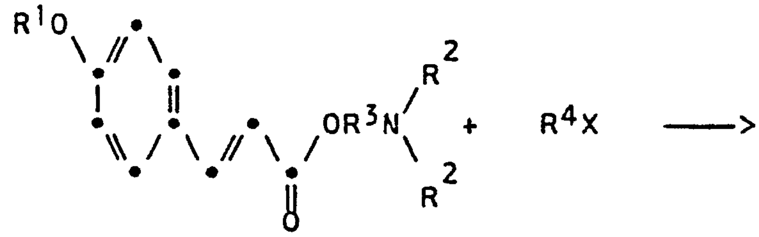

II III

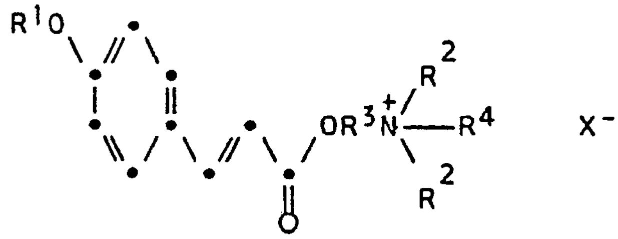

I

wherein $R^1$, $R^2$, $R^3$, $R^4$, and $X^-$ are as defined above.

In the preferrred procedure, the tertiary amine and the alkylating agent are brought together to react in approximately a 1:1 molar ratio. The reaction may be carried out in the presence or absence of solvent and at ambient or elevated temperature. The use of a polar solvent at reflux to effect the alkylation is generally preferred. Especially preferred polar solvents are alcoholic solvents such as methanol, ethanol, isopropanol and the like. If desired, the crude products may be further purified by recrystallization from appropriate solvents.

Compounds of formula II may be readily prepared by any of a number of processes known to those skilled in the art, i.e., condensation of activated cinnamic acid derivatives with dialkylamino alcohols. [See for example Jung et al., supra.]

The novel salts of this invention, i.e., those salts of formula I wherein $R^4$ is an alkyl group of from sixteen to twenty-two carbon atoms, all possess superior substantivity properties. For economic reasons such as availability of starting materials, it is preferred, however, to use a novel salt wherein $R^1$ is a methyl group, $R^2$ is a methyl or ethyl group, $R^3$ is ethylene, propylene or butylene and the halogen anion is either bromide or chloride. Although substantivity appears to increase markedly as length of the $R^4$ substituent increases (all other substituents being held constant) those salts wherein $R^4$ is hexadecyl or octadecyl are also preferred for economic reasons with the octadecyl being particularly preferred because of its superior substantivity. It is especially preferred to use an octadecyl salt wherein $R^2$ is methyl and $R^3$ is ethylene, as these salts appear to offer superior substantivity properties to those salts in which $R^2$ is ethyl and/or $R^3$ is propylene or butylene.

The novel salts of formula I may be used as sunscreen agents, e.g. in compositions for the protection of hair, fabric and skin from the deleterious effects of the sun. They may be effectively incorporated in concentrations of from about 0.1 to about 10.0% by weight into aqueous, alcoholic or hydroalcoholic solutions, suspensions or gels, as well as into emulsified lotions, creams, milks, aerosols, gels and the like by methods well known in the art. When used in topical preparations for protection of the skin such as sunscreen lotions, lip balms, sticks and gels, it is preferred to use a salt of formula I in a range from about 2% to about 10% by weight. In skin care preparations such as hand and body creams, facial creams, facial makeup, after shaves, colognes and astringents, it is preferred to use from about 0.1% to about 5% by

weight. In compositions for the protection of hair such as shampoos, conditioners, styling mousses, styling gels and glazes, it is also preferred to use from about 0.1% to about 5% by weight. Fabric treatment formulations such as liquid and solid fabric softeners and detergents may contain from about 0.1% to about 1.0% of a compound of formula I.

A number of examples are provided herein to illustrate the preferred embodiments of this invention. Included are examples directed to the synthesis of the novel compounds of this invention and examples directed to the utility of these compounds. The examples provided herein are included for the sole purpose of illustrating the preferred embodiments and should not be construed as limiting. They are intended to embrace any equivalents or obvious extensions which are known or should be known to a person skilled in the art.

EXAMPLE 1: PREPARATION OF QUATERNARY SALTS

A) 2-(N,N-Dimethyl-N-n-hexadecylamino)ethyl 3-(4-methoxyphenyl)-2-propenoate bromide

A mixture of 2-(N,N-dimethylamino)ethyl 3-(4-methoxyphenyl)-2-propenoate (50.0g; 0.20 mole) and 1-bromohexadecane (61.12g; 0.20 mole) was heated to 100° C for 0.5h. The resultant mass was finely pulverized, triturated with diethylether and dried. The product (93.1g; 84.0%) was obtained as an off-white solid: m.p. 112-123° C. Spectral data ($^1$H NMR, IR and UV) were consistent with the assigned structure.

B) 2-(N,N-Dimethyl-N-n-octadecylamino)ethyl 3-(4-methoxyphenyl)-2-propenoate bromide

A mixture of 2-(N,N-dimethylamino)ethyl 3-(4-methoxyphenyl)-2-propenoate (24.9g; 0.10 mole), isopropanol (50ml), and 1-bromooctadecane (33.3g; 0.10 mole) was allowed to reflux under a dry atmosphere for 5h. The hot reaction mixture was diluted with toluene (125ml) and cooled to 0° C. The product (38.8g; 66.7%) was filtered and dried. A white solid was obtained: m.p. 162-164° C. Spectral data ($^1$H NMR, IR, and UV) were consistent with the assigned structure.

The wavelengths of maximum absorption ($\lambda$ max) in ethanol, and the intensity of the absorption calculated as molar extinction coefficient ($\epsilon$ max) for the above salts can be found below in Table 1 of Example 2. Table 1 also gives data for other, similarly prepared salts of this invention.

EXAMPLE 2: SUBSTANTIVITY TO HAIR

The substantivity of the novel salts of formula I to hair was tested in the following manner. A 0.01% w/v solution (0.01g of sunscreen per 100ml of solution) of each sunscreen to be evaluated was prepared in 50% aqueous ethanol. A swatch of virgin, brunette hair weighing 0.10g was immersed into 10ml of the sunscreen solution and allowed to stand undisturbed at room temperature for 30 min. The solution was decanted away from the hair, and the hair shaken free of excess liquid and transferred to a clean, dry beaker, where it was allowed to air-dry overnight. Following this, the hair was allowed to stand undisturbed in 10ml of distilled water for 30 min. at room temperature, then was transferred to a clean beaker and redried overnight. Finally, the swatch was soaked in 10ml of absolute ethanol for 30 min.; an analysis of this ethanol wash by UV spectrophotometry indicated the extent to which the sunscreen remained bound onto the hair following the aqueous soak. A blank control using 50% aqueous ethanol in place of the initial sunscreen solution confirmed that no inherent UV-absorbing material was leached from the hair by the ethanol wash and that all the UV absorption was due to the absorber which had adhered to the hair as a result of this experiment. The substantivity of the sunscreen can be calculated as follows:

Sunscreen Concentration (mg/ml) in ethanol wash =

$$\frac{(\text{mol. wt. sunscreen})(\text{absorbance of solution})}{(\varepsilon\ \text{max[ethanol] of sunscreen})}$$

Thus, total mg sunscreen per g hair =

$$\frac{(\text{total ml sol.})(\text{mol.wt. sunscreen})(\text{absorbance of solution})}{(\varepsilon\ \text{max[ethanol] of sunscreen})(\text{total wt. hair, g})}$$

$$\text{absorbance} = \text{extinction} = \log\frac{Io}{I} = \varepsilon \bullet c \bullet d.$$

Table 1 gives the results found for the novel salts of formula I prepared as illustrated in Example 1.

TABLE 1

| COMPOUND | λ MAX | ε MAX | mg SUNSCREEN/ g HAIR |
|---|---|---|---|
| 2-(N,N-Dimethyl-N-n-hexadecylamino)ethyl 3-(4-methoxyphenyl)-2-propenoate bromide (a) | 316 | 24,500 | 0.794 |
| 2-(N,N-Dimethyl-N-n-octadecylamino)ethyl 3-(4-methoxyphenyl)-2-propenoate bromide (b) | 316 | 25,700 | 1.076 |
| 2-(N,N-Dimethyl-N-n-hexadecylamino)ethyl 3-(4-methoxyphenyl)-2-propenoate chloride (c) | 314 | 25,600 | 0.495 |
| 2-(N,N-Diethyl-N-n-octadecyl-amino)ethyl 3-(4-methoxyphenyl)-2-propenoate bromide (c) | 314 | 29,100 | 0.507 |
| 3-(N,N-Diethyl-N-n-eicosylamino)-1-propyl 3-(4-methoxyphenyl)-2-propenoate bromide (c) | 312 | 21,200 | 1.457 |
| 2-(N,N-Dimethyl-N-n-docosylamino)-ethyl 3-(4-methoxyphenyl)-2-propenoate bromide (c) | 316 | 22,200 | 2.396 |

(a) Example 1A.
(b) Example 1B.
(c) Novel compounds of formula I prepared in a manner similar to Example 1.

Table 1 demonstrates that the novel salts of this invention are highly substantive to hair.

The superior substantivity properties of those salts wherein $R^4$ is an alkyl group of from sixteen to twenty-two carbon atoms is graphically illustrated in figure I. In figure I, the substantivities (mg sunscreen/g hair) have been plotted against the number of carbon atoms in the $R^4$ alkyl group for a series of novel compounds of formula I wherein all the substituents are held constant ($R^1$ and $R^2$ are methyl, $R^3$ is ethyl and $X^-$ is bromide) except $R^4$, which varies from propyl (three carbons) to docosyl (twenty two carbons).

FIGURE I

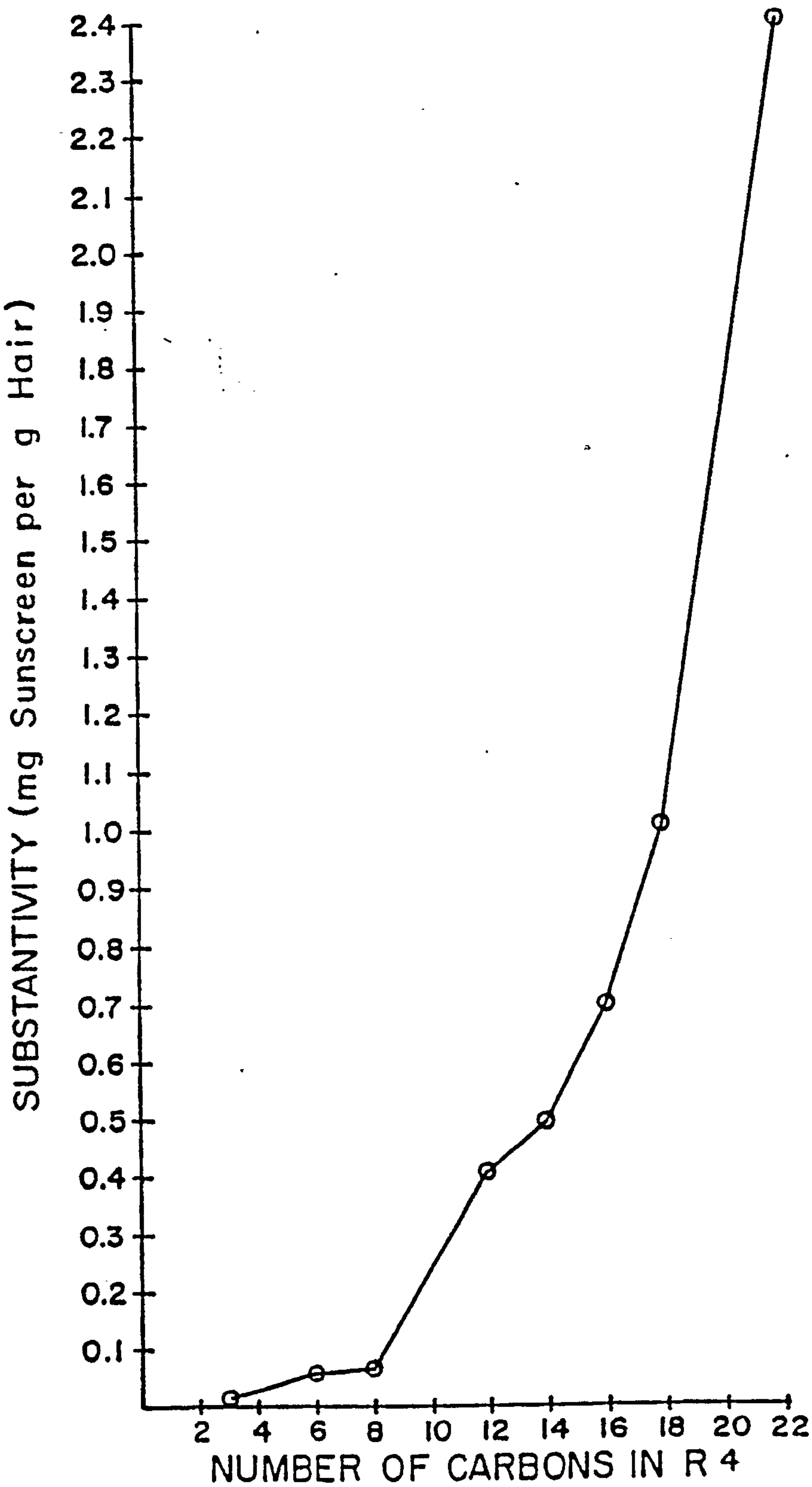


EXAMPLE 3: SUBSTANTIVITY TO COTTON

The substantivity of the novel salts of formula I to cotton was tested in the following manner. A 4.0%

w/v solution of the salt prepared in Example 1B was prepared in 50% aqueous isopropanol at room temperature. Approximately 0.2ml of the sunscreen solution was pipetted onto commercial 100% cotton toweling, which had been previously laundered and treated with brightening agents. The solution, which saturated a 1 inch diameter circle of fabric, was dried in a stream of hot air for several minutes. The presence of sunscreen on the fabric was readily confirmed by an examination of the fabric under shortwave UV light, viz., the sunscreen appeared dark blue or blue-black against a bright blue fluorescent background. The towel sample was allowed to stand undisturbed in a bath of distilled water at 50-55° C for 8 min., then was removed and redried in a hot air stream. A reexamination of the fabric under UV light revealed essentially complete retention of the sunscreen by the fabric material. Similar results were found for the other salts of formula I listed in Table 1.

EXAMPLE 4: APPLICATIONS

The following examples illustrate the use of the novel salts in aqueous and hydroalcoholic systems as well as in oil/water emulsions.

A) Rinse Out Conditioner - oil/water emulsion

| Part I | % by weight |
|---|---|
| Water | q.s. to 100 |
| Standamol Conc. 1002™ (Henkel) (Cetearyl alcohol (mixture of fatty alcohols, predominantly cetyl/ stearyl alcohols)/PEG-40 hydrogenated castor oil (polyethylene glycol derivatives of hydrogenated castor oil with an average of 40 moles of ethylene oxide)) | |
| Stearalkonium chloride (benzyl dimethyl stearyl ammonium chloride) | 3.00 |
| Ceteth-2 (polyoxyethylene (2) cetyl ether having the formula $CH_3(CH_2)_{14}CH_2(OCH_2CH_2)_nOH$, $\bar{n} = 2$) | 2.00 |
| Propylene glycol | 1.00 |
| Stearalkonium chloride | 3.00 |
| Methyl paraben (4-hydroxy benzoic acid methyl ester) | 0.10 |
| 2-(N,N-Dimethyl-N-n-docosylamino)-ethyl 3-(4-methoxyphenyl)-2-propenoate bromide | 0.50 |

| Part II | |
|---|---|
| Panthenol | 0.50 |
| Kathon CG™ (Rohm and Haas) (2-Methyl-5-chloroisothiazolinone/ 2-methylisothiazolinone) | 0.03 |
| Citric acid | 0.02 |
| Water | 10.00 |

Part III

| | |
|---|---|
| Fragrance | q.s. |

The ingredients of Part I are blended at 70° C until uniform. The ingredients of Part II are blended at room temperature until all solids have been dissolved. Part II is then added to Part I with mixing until homogeneous. The product is cooled with mixing and perfumed at 40° C.

B) Styling Mousse - hydroalcoholic aerosol

| Part I | % by weight |
|---|---|
| Polyquaternium-4 (2% in water) (a copolymer of hydroxyethylcellulose and diallyldimethylammonium chloride) | 50.00 |
| Quaternium-26 (minkamidopropyl dimethyl 2-hydroxyethyl ammonium chloride having the formula $[RCONH(CH_2)_3N(CH_3)_2CH_2CH_2OH]\ Cl^-$, where R represents the fatty acid groups derived from mink oil) | 3.50 |
| Glycerin | 7.00 |
| Water | q.s. to 100 |
| 2-(N,N-Dimethyl-N-n-octadecylamino)ethyl 3-(4-methoxyphenyl)-2 propenoate bromide | 1.00 |

Part II

| | |
|---|---|
| Alcohol | 20.00 |
| PVP/VA copolymer (a copolymer of vinyl acetate and vinylpyrrolidone monomer having the formula $(C_6H_9NO \bullet C_4H_6O_2)_x$ | 2.00 |
| PPG-12 PEG-50 lanolin (polyoxypropylene (50) polyoxyethylene (12) lanolin having the formula $R(OCH(CH_3(CH_2)_x(OCH_2CH_2)_yOH$; R = lanolin radical, $\bar{x} = 12$, $\bar{y} = 50$) | 0.50 |
| Fragrance | q.s. |

The ingredients of Parts I and II are mixed separately in the order listed. Part I is then added to Part II with thorough mixing. The resultant mixture is filled into an appropriate aerosol container and diluted to 94.00% with propellant A-46.

C) Fabric Softener - an aqueous system

| Component | % by weight |
|---|---|
| Water | q.s. to 100 |
| Quaternium-27 (an imidazole methosulfate derivative of tallow acid amide having the formula 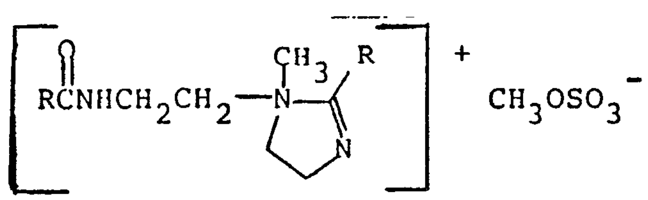 where R is derived from the tallow acid radical) | 6.00 |
| Fragrance | 0.20 |
| 2-(N,N-Dimethyl-N-n-hexadecylamino)ethyl 3-(4-methoxyphenyl)-2-propenoate bromide | 1.00 |

The quaternium-27 and water are each heated to 50° C, and combined with mixing. The quaternary bromide is added and the mixture is allowed to cool. The product is perfumed at 40° C.

D) Daily Use Cream - an oil/water emulsion

| Part I | % by weight |
|---|---|
| Water | q.s. to 100 |
| Propylene glycol | 5.00 |
| Methyl paraben | 0.20 |
| 2-N,N-Dimethyl-N-n hexadecylamino)ethyl 3-(4-methoxyphenyl)-2-propenoate chloride | 3.00 |

| Part II | |
|---|---|
| Mineral oil | 10.00 |
| Arlacel 165™ (ICI Americas) (glyceryl stearate having the formula: $CH_3(CH_2)_{16}COOCH_2CH(OH)CH_2OH$/ PEG-100 stearate (polyoxyethylene (100) monostearate of the formula: $CH_3(CH_2)_{16}COOCH_2CH)_nOH$, $\overline{n}$ = 100)) | 8.00 |
| Cetyl alcohol | 4.00 |
| Stearic acid | 3.00 |
| Stearyl alcohol | 1.00 |
| Jojoba oil | 0.50 |
| Propyl paraben | 0.10 |

| Part III | |
|---|---|
| Fragrance | q.s. |

Parts I and II are heated separately to 80° C. When both phases are clear and uniform, Part II is added to Part I with continuous mixing. The product is cooled with mixing and perfumed at 40° C.

E) Sunscreen Lotion - an oil/water emulsion

| Part I | % by weight |
|---|---|
| Water | q.s. to 100 |
| Glycerin | 10.00 |
| Methyl paraben | 0.25 |
| Citric acid | 0.17 |
| 2-(N,N-Dimethyl-N-n-octadecylamino)ethyl 3-(4-methoxyphenyl)-2-propenoate bromide | 2.00 |

| Part II | |
|---|---|
| Caprylic/capric triglyceride | 3.00 |
| Isopropyl palmitate | 2.00 |
| Mineral oil | 1.00 |
| Emulgade 1000 Ni™ (Henkel) (Cetearyl alcohol/ceteareth-20 [polyoxy-ethylene (20) cetyl/stearyl ether: $R(OCH_2CH_2)_nOH$, R = cetyl/stearyl blend, $\bar{n}$ = 20)] | 2.00 |

| Part II Con't | |
|---|---|
| Tocopheryl acetate | 0.50 |
| Glyceryl stearate | 2.00 |
| Stearamidopropyl dimethylamine | 1.00 |
| Dimethicone (a mixture of fully methylated linear siloxane polymers end-blocked with trimethylsiloxy units: $(CH_3)_3SiO[Si(CH_3)_2-O]_nSi(CH_3)_3$) | 0.20 |
| Propyl paraben (4-hydroxy benzoic acid propyl ester) | 0.10 |

| Part III | |
|---|---|
| Fragrance | q.s. |

Parts I and II are heated separately to 80 °C. When both phases are clear and uniform, Part II is added

to Part I with continuous mixing. The product is cooled with mixing and perfumed at 40 ° C.

## Claims

Claims For The Contracting States AT BE CH DE FR GB IT LI NL :

1. A compound of the formula

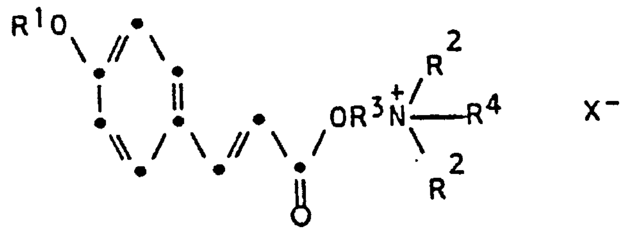

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is a halogen anion.

2. A compound according to claim 1 wherein $R^1$ is a methyl group; $R^2$ is a methyl or ethyl group; $R^3$ is ethylene, propylene or butylene; and $X^-$ is bromide or chloride.

3. A compound according to claim 1 or 2 wherein $R^4$ is an alkyl group containing sixteen, eighteen, twenty or twenty-two carbon atoms.

4. The compound according to claim 1, 2 or 3 wherein $R^2$ methyl; $R^3$ ethylene; and $R^4$ is an alkyl group containing sixteen or eighteen carbon atoms.

5. The compound according to claim 1, 2, 3 or 4 wherein $R^4$ is n-octadecyl; and $X^-$ is bromide.

6. An ultraviolet light absorbing composition comprising a compound of the formula

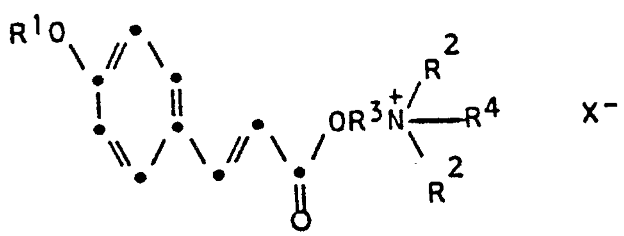

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is a halogen anion,
in a suitable carrier, said compound being present in an effective ultraviolet absorbing amount.

7. Process for the manufacture of compounds of formula

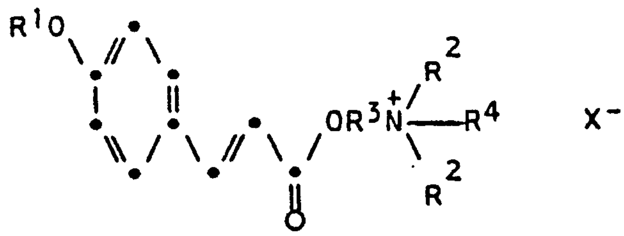

I

wherein $R^1$ is an alkyl group containing from one to four carbon atoms; $R^2$ is an alkyl group containing from one to s1x carbon atoms; $R^3$ is an alkylene group containing from two to six carbon atoms; $R^4$ is an alkyl group containing from sixteen to twenty-two carbon atoms; and $X^-$ is a halogen anion, comprising reacting an amine of formula

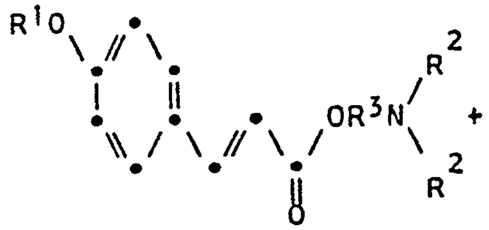

II

with the alkylating agent

$$R^4X \qquad III$$

wherein $R^1$ to $R^4$ and X are as above.

8. A method for protecting skin, hair or fabric from the effects of ultraviolet radiation, which comprises applying to said skin, hair or fabric an effective ultraviolet absorbing amount of a compound of the formula

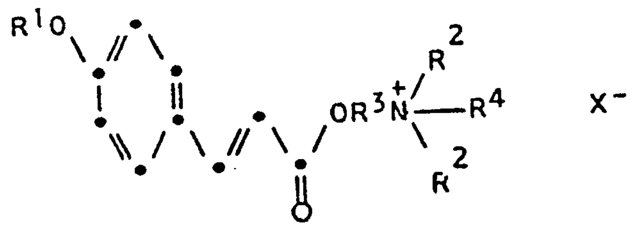

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is a halogen anion, in a carrier suitable for application to said skin, hair or fabric.

9. Use of the compounds of the formula

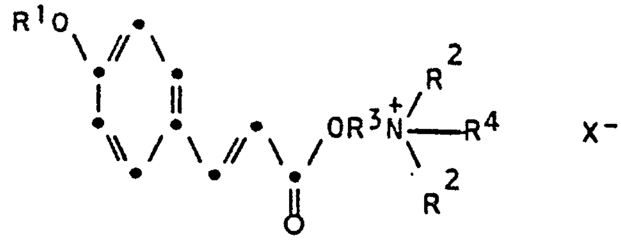

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is a halogen anion, as ultraviolet absorbing agents.

Claim for the Contracting State ES :

1. Process for the manufacture of compounds of formula

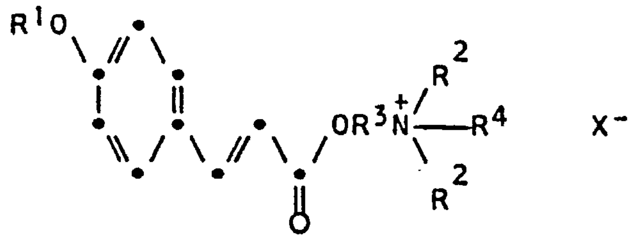

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is a halogen anion, comprising reacting an amine of formula

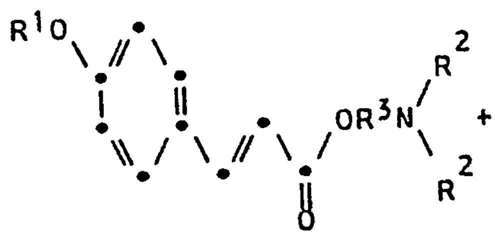

II

with the alkylating agent

$$R^4X \qquad III$$

wherein $R^1$ to $R^4$ and $X^-$ are as above.

2. An ultraviolet light absorbing composition comprising a compound of the formula

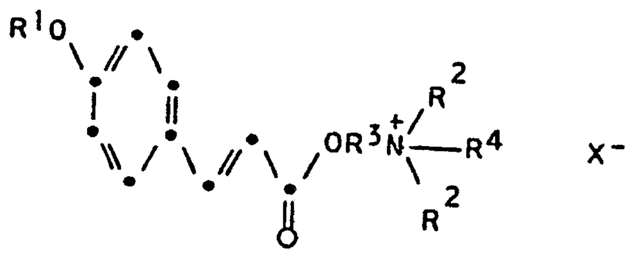

I

wherein $R^1$ is an alkyl group containing from one to four carbon atoms; $R^2$ is an alkyl group containing from one to six carbon atoms; $R^3$ is an alkylene group containing from two to six carbon atoms; $R^4$ is an alkyl group containing from sixteen to twenty-two carbon atoms; and $X^-$ is halogen anion, in a suitable carrier, said compound being present in an effective ultraviolet absorbing amount.

3. An ultraviolet light absorbing composition according to claim 2, wherein $R^1$ is a methyl group; $R^2$ is a methyl or ethyl group, $R^3$ is ethylene, propylene or butylene; and $X^-$ is bromide or chloride.

4. An ultraviolet light absorbing composition according to claim 2, wherein $R^4$ is an alkyl group containing sixteen, eighteen, twenty or twenty-two carbon atoms.

5. An ultraviolet light absorbing ccmposition according to claim 2, wherein $R^2$ methyl; $R^3$ ethylene; and $R^4$ is an alkyl group containing sixteen or eighteen carbon atoms.

6. An ultraviolet light absorbing composition according to claim 2, wherein $R^4$ is n-octadecyl; and $X^-$ is bromide.

**Revendications**

REVENDICATIONS pour les Etats Contractants : AT, BE, CH, DE, FR, GB, IT, LI, NL

1. Composé de formule

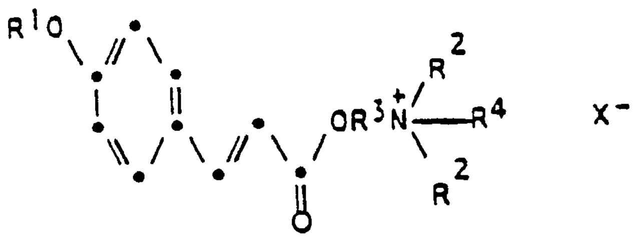

I

dans laguelle $R^1$ est un groupe alkyle contenant de 1 à 4 atomes de carbone ; $R^2$ est un groupe alkyle contenant de 1 à 6 atomes de carbone ; $R^3$ est un groupe alkylène contenant de 2 à 6 atomes de carbone ; $R^4$ est un groupe alkyle contenant de 16 à 22 atomes de carbone ; et $X^-$ est un anion halogène.

2. Composé selon la revendication 1, dans leguel $R^1$ est un groupe méthyle ; $R^2$ est un groupe méthyle ou éthyle ; $R^3$ est un groupe éthylène, propylène ou butylène ; et $X^-$ est un anion bromure ou chlorure.

3. Composé selon la revendication 1 ou 2, dans leguel $R^4$ est un groupe alkyle contenant 16, 18, 20 ou 22 atomes de carbone.

4. Composé selon l'une quelconque des revendications 1, 2 ou 3, dans lequel $R^2$ est un groupe méthyle ; $R^3$ est un groupe éthylène ; et $R^4$ est un groupe alkyle contenant 16 ou 18 atomes de carbone.

5. Composé selon l'une quelconque des revendications 1, 2, 3 ou 4, dans lequel $R^4$ est un groupe n-octadécyle ; et $X^-$ est un anion bromure.

6. Composition absorbant la lumière ultraviolette comprenant un composé de formule

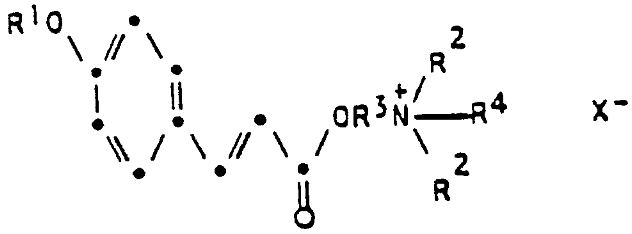

I

dans laquelle $R^1$ est un groupe alkyle contenant de 1 à 4 atomes de carbone ; $R^2$ est un groupe alkyle contenant de 1 à 6 atomes de carbone ; $R^3$ est un groupe alkylène contenant de 2 à 6 atomes de carbone ; $R^4$ est un groupe alkyle contenant de 16 à 22 atomes de carbone ; et $X^-$ est un anion halogène, dans un véhicule approprié, ledit composé étant présent en une quantité efficace pour absorber les ultraviolets.

7. Procédé pour la préparation de composés de formule

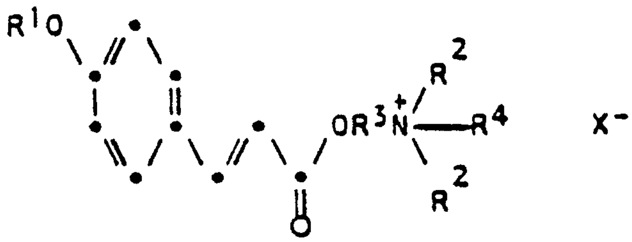

I

dans laquelle $R^1$ est un groupe alkyle contenant de 1 à 4 atomes de carbone ; $R^2$ est un groupe alkyle contenant de 1 à 6 atomes de carbone ; $R^3$ est un groupe alkylène contenant de 2 à 6 atomes de carbone ; $R^4$ est un groupe alkyle contenant de 16 à 22 atomes de carbone ; et $X^-$ est un anion halogène, comprenant la réaction d'une amine de formule

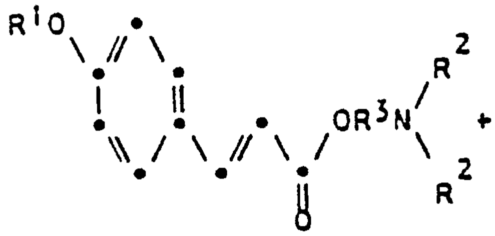

II

avec l'agent d'alkylation

$R^4X$ III

dans laquelle $R^1$ à $R^4$ et X sont comme ci-dessus.

8. Procédé pour protéger la peau, les cheveux ou un textile des effets du rayonnement ultraviolet, qui comprend l'application à la peau, aux cheveux ou au textile d'une quantité efficace pour absorber les ultraviolets d'un composé de formule

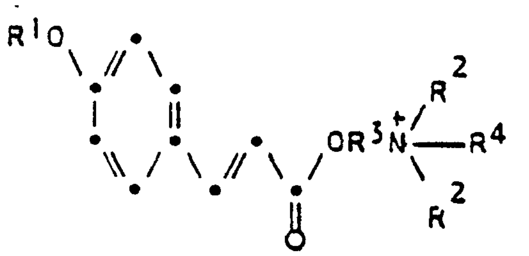

$X^-$ I

dans laquelle $R^1$ est un groupe alkyle contenant de 1 à 4 atomes de carbone ; $R^2$ est un groupe alkyle contenant de 1 à 6 atomes de carbone ; $R^3$ est un groupe alkylne contenant de 2 à 6 atomes de carbone ; $R^4$ est un groupe alkyle contenant de 16 à 22 atomes de carbone ; et $X^-$ est un anion halogène, dans un véhicule convenant à l'application à la peau, au cheveux ou au textile.

9. Utilisation des composés de formule

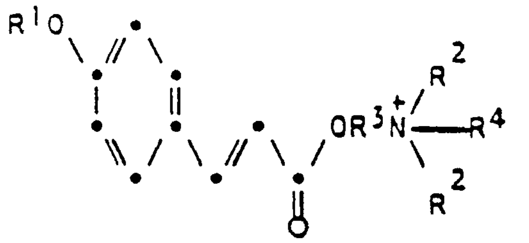

$X^-$ I

dans laquelle $R^1$ est un groupe alkyle contenant de 1 à 4 atomes de carbone ; $R^2$ est un groupe alkyle contenant de 1 à 6 atomes de carbone ; $R^3$ est un groupe alkylène contenant de 2 à 6 atomes de carbone ; $R^4$ est un groupe alkyle contenant de 16 à 22 atomes de carbone ; et $X^-$ est un anion halogène, comme agents absorbant les ultraviolets.

Revendications pour l'etat Contractant : ES

1. Procédé pour la préparation de composés de formule

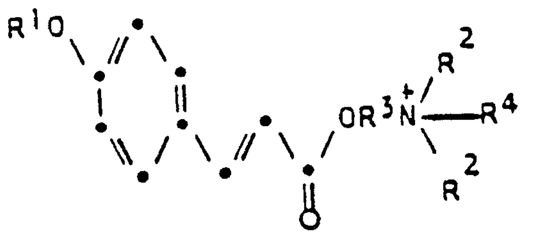

$X^-$ I

dans laquelle $R^1$ est un groupe alkyle contenant de 1 à 4 atomes de carbone ; $R^2$ est un groupe alkyle contenant de 1 à 6 atomes de carbone ; $R^3$ est un groupe alkylène contenant de 2 à 6 atomes de carbone ; $R^4$ est un groupe alkyle contenant de 16 à 22 atomes de carbone ; et $X^-$ est un anion halogène,
comprenant la réaction d'une amine de formule

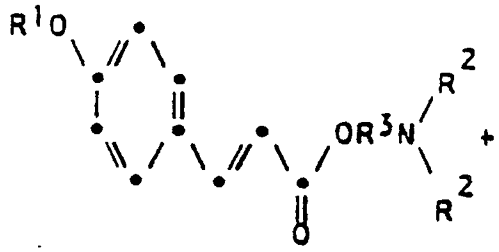

II

avec l'agent d'alkylation

$R^4X$ III

dans laquelle $R^1$ à $R^4$ et X sont comme ci-dessus.

2. Composition absorbant la lumière ultraviolette comprenant un composé de formule

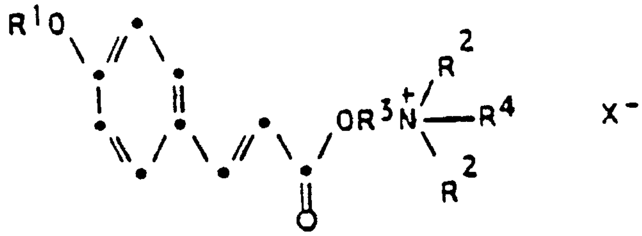

I

dans laquelle $R^1$ est un groupe alkyle contenant de 1 à 4 atomes de carbone ; $R^2$ est un groupe alkyle contenant de 1 à 6 atomes de carbone ; $R^3$ est un groupe alkylène contenant de 2 à 6 atomes de carbone ; $R^4$ est un groupe alkyle contenant de 16 à 22 atomes de carbone ; et $X^-$ est un anion halogène, dans un véhicule approprié, ledit composé étant présent en une quantité efficace pour absorber les ultraviolets.

3. Composition absorbant la lumière ultraviolette selon la revendication 2, dans laquelle $R^1$ est un groupe méthyle ; $R^2$ est un groupe méthyle ou éthyle ; $R^3$ est un groupe éthylène, propylène ou butylène ; et $X^-$ est un anion bromure ou chlorure.

4. Composition absorbant la lumière ultraviolette selon la revendication 2, dans laquelle $R^2$ est un groupe alkyle contenant 16, 18, 20 ou 22 atomes de carbone.

5. Composition absorbant la lumière ultraviolette selon la revendication 2, dans laquelle $R^2$ est un groupe méthyle ; $R^3$ est un groupe éthylène ; et $R^4$ est un groupe alkye contenant 16 ou 18 atomes de carbone.

6. Composition absorbant la lumière ultraviolette selon la revendication 2, dans laquelle $R^4$ est un groupe n-octadécyle ; et $X^-$ est un anion bromure.

## Ansprüche

Patentansprüche für die benannten Vertragsstaaten : AT, BE, CH, DE, FR, GB, IT, LI, NL

1. Eine Verbindung der Formel

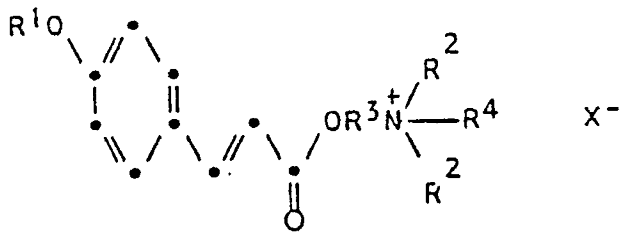

I

worin $R^1$ $C_{1-4}$-Alkyl, $R^2$ $C_{1-6}$-Alky1, $R^3$ $C_{2-6}$-Alkylen, $R^4$ $C_{16-22}$-Alkyl und $X^-$ ein Halogenidanion bedeuten.

2. Eine Verbindung gemäss Anspruch 1, worin $R^1$ Methyl, $R^2$ Methyl oder Ethyl, $R^3$ Ethylen, Propylen oder Butylen und $X^-$ Bromid oder Chlorid sind.

3. Eine Verbindung gemäss Anspruch 1 oder 2, worin $R^4$ ein Alkylrest mit 16, 18, 20 oder 22 Kohlenstoffatomen ist.

4. Eine Verbindung gemäss Anspruch 1,2 oder 3, worin $R^2$ Methyl, $R^3$ Ethylen und $R^4$ ein Alkylrest mit 16 oder 18 Kohlenstoffatomen sind.

5. Eine Verbindung gemäss Anspruoh 1, 2, 3 oder 4, worin $R^4$ n-Oktadecyl und $X^-$ Bromid sind.

6. Eine das ultraviolette Licht absorbierende Komposition, enthaltend eine Verbindung

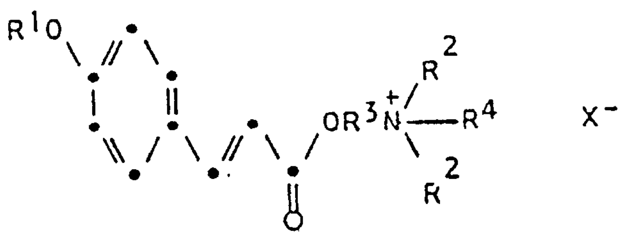

I

worin $R^1$ $C_{1-4}$-Alkyl, $R^2$ $C_{1-6}$-Alkyl, $R^3$ $C_{2-6}$-Alkylen, $R^4$ $C_{16-22}$-Alkyl und $X^-$ ein Halogenidanion bedeuten, in einem geeigneten Träger in einer zur Absorption des ultravioletten Lichts wirksamen Menge.

7. Verfahren zur Herstellung der Verbindungen der Formel

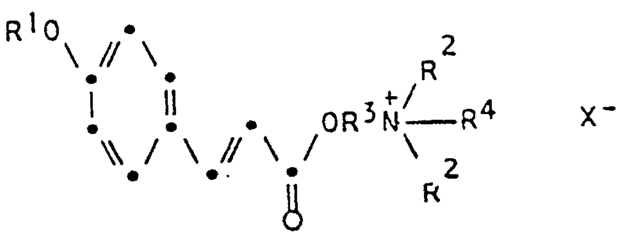

I

worin $R^1$ $C_{1-4}$-Alkyl, $R^2$ $C_{1-6}$-Alkyl, $R^3$ $C_{2-6}$-Alkylen, $R^4$ $C_{16-22}$-Alkyl und $X^-$ ein Halogenidanion bedeuten, dadurch gekennzeichnet, dass man ein Amin der Formel

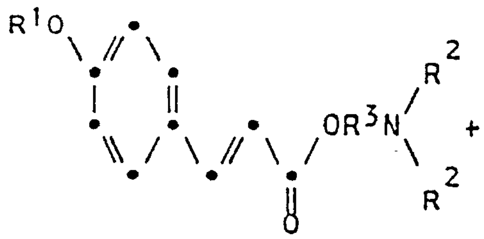

II

mit einem Alkylierungsmittel

$$R^4X \qquad III$$

worin $R^1$ bis $R^4$ und X die obigen Bedeutungen besitzen, umsetzt.

8. Verfahren zum Schutz der Haut, des Haares oder von Geweben vor der Wirkung der ultravioletten Strahlung, dadurch gekennzeichnet, dass man zu diesem Zweck die Haut, das Haar oder das Gewebe mit einer wirksamen Menge einer Verbindung der Formel

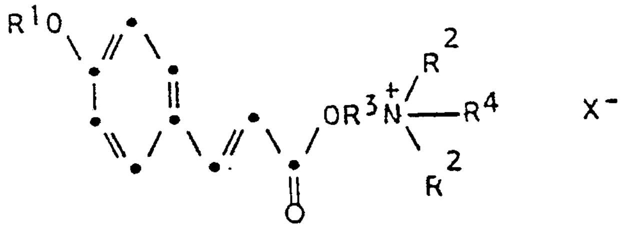

I

worin $R^1$ $C_{1-4}$-Alkyl, $R^2$ $C_{1-6}$-Alkyl, $R^3$ $C_{2-6}$-Alkylen, $R^4$ $C_{16-22}$-Alkyl und $X^-$ ein Halogenidanion bedeuten, in einem zur Applikation auf Haut, Haar oder Gewebe geeigneten Träger behandelt.

9. Verwendung der Verbindungen der Formel

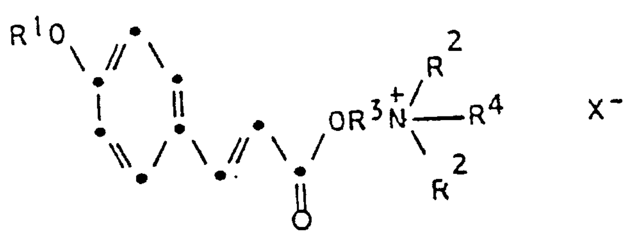

I

worin $R^1$ $C_{1-4}$-Alkyl, $R^2$ $C_{1-6}$-Alkyl, $R^3$ $C_{2-6}$-Alkylen, $R^4$ $C_{16-22}$-Alkyl und $X^-$ ein Halogenidanion bedeuten, als das ultraviolette Licht absorbierende Agentien.

Patentansprüche Für den benannten Vertragsstaat : ES

1. Verfahren zur Herstellung der Verbindungen der Formel

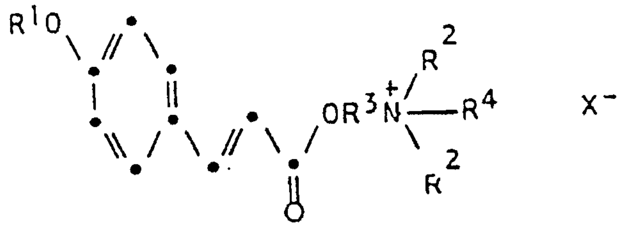

I

worin $R^1$ $C_{1-4}$-Alkyl, $R^2$ $C_{1-6}$-Alkyl, $R^3$ $C_{2-6}$-Alkylen, $R^4$ $C_{16-22}$-Alkyl und $X^-$ ein Halogenidanion bedeuten, dadurch gekennzeichnet, dass man ein Amin der Formel

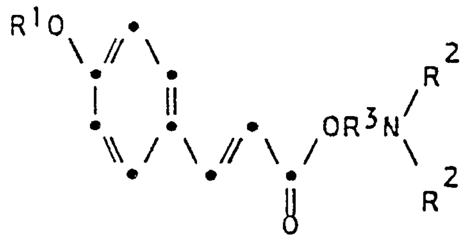

+ II

mit einem Alkylierungsmittel

$R^4X$ III

worin $R^1$ bis $R^4$ und X die obigen Bedeutungen besitzen, umsetzt.

2. Eine das ultraviolette Licht absorbierende Komposition, enthaltend eine Verbindung

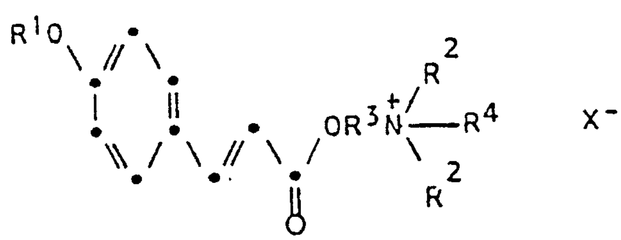

I

worin $R^1$ $C_{1-4}$-Alkyl, $R^2$ $C_{1-6}$-Alkyl, $R^3$ $C_{2-6}$-Alkylen, $R^4$ $C_{16-22}$-Alkyl und $X^-$ ein Halogenidanion bedeuten, in einem geeigneten Träger in einer zur Absorption des ultravioletten Lichts wirksamen Menge.

3. Eine das ultraviolette Licht absorbierende Komposition gemäss Anspruch 2,worin $R^1$ Methyl, $R^2$ Methyl oder Ethyl, $R^3$ Ethylen, Propylen oder Butylen und $X^-$ Bromid oder Chlorid sind.

4. Eine das ultraviolette Licht absorbierende Komposition gemäss Anspruch 2, worin $R^4$ ein Alkylrest mit 16, 18, 20 oder 22 Kohlenstoffatomen ist.

5. Eine das ultraviolette Licht absorbierende Komposition gemäss Anspruch 2, worin $R^2$ Methyl, $R^3$ Ethylen und $R^4$ ein Alkylrest mit 16 oder 18 Kohlenstoffatomen sind.

6. Eine das ultraviolette Licht absorbierende Komposition gemäss Anspruch 2, worin $R^4$ n-Oktadecyl und

$X^-$ Bromid sind.